# EUROPEAN PATENT SPECIFICATION

(11) **EP 1 052 963 B1**
(45) Date of publication and mention of the grant of the patent: **23.08.2006**
(21) Application number: 99905970.2
(22) Date of filing: 12.02.1999
(51) Int. Cl.: A61K 31/19, A61P 17/12, A61P 31/10

(54) **USE OF ALKANOIC ACID CONTAINING COMPOSITIONS FOR TREATING NAIL FUNGAL CONDITIONS**
VERWENDUNG VON ALKANSÄUREN-ENTHALTENDEN ZUSAMMENSETZUNGEN ZUR BEHANDLUNG VON NAGELPILZ-ERKRANKUNGEN
UTILISATION DE COMPOSITIONS CONTENANT DES ACIDES ALCANOIQUES POUR TRAITER DES MYCOSES ONGULAIRES

(30) Priority: 13.02.1998 US 23449; 19.05.1998 US 81256; 14.10.1998 US 172461
(43) Date of publication of application: 22.11.2000
(60) Divisional application: 06013639.7
(73) Proprietor: Buck, Carol J., Princeton, NJ 08540 (US)
(72) Inventor: Buck, Carol J., Princeton, NJ 08540 (US)
(74) Representative: Lawrence, John
(86) International application number: PCT/US1999/003169
(87) International publication number: WO 1999/040888

(56) References cited:
- EP-A- 0 011 511
- EP-A- 0 742 004
- EP-A- 0 755 676
- WO-A-00/32157
- WO-A-87/04617
- WO-A-88/04921
- WO-A-93/00882
- WO-A-96/11572
- WO-A-97/17953
- WO-A-97/46231
- WO-A-99/20250
- DE-A- 19 536 423
- GB-A- 745 179
- GB-A- 756 002
- US-A- 2 729 586
- US-A- 3 482 581
- US-A- 4 363 815
- US-A- 4 871 535
- US-A- 5 091 171
- US-A- 5 304 370
- US-A- 5 462 714
- US-A- 5 635 168
- DATABASE WPI Week 199133, 5 July 1991 (1991-07-05) Derwent Publications Ltd., London, GB; AN 1991-242242 XP002229541 SHIRANE MIYAKO ET AL.: "Cosmetic for common acne" & JP 03 157311 A (KANEBO LTD), 5 July 1991 (1991-07-05)
- MONTANA J B ET AL: "A DOUBLE-BLIND, VEHICLE-CONTROLLED STUDY OF THE SAFETY AND EFFICACYOF FUNGOID TINCTURE IN PATIENTS WITH DISTAL SUBUNGUAL ONYCHOMYCOSIS OF THE TOES" CUTIS, EXCERPTA MEDICA, BELLE MEAD,NJ, US, vol. 53, no. 6, 1 June 1994 (1994-06-01), pages 313-316, XP002070145 ISSN: 0011-4162
- DATABASE WPI Week 198821, 13 April 1988 (1988-04-13) Derwent Publications Ltd., London, GB; AN 1988-142664 XP002229542 SHIMURA KAZUMI: "Remedy for dermatosis" & JP 63 083026 A (SHIMURA KAZUMI), 13 April 1988 (1988-04-13)
- DATABASE BIOSIS [Online] BIOSCIENCES INFORMATION SERVICE, PHILADELPHIA, PA, US; 1990 HASHIMOTO Y ET AL: "TREATMENT OF ACNE VULGARIS AND ACNE CONGLOBATA WITH 20 PERCENT ADIPIC ACID CREAM" Database accession no. PREV199191036411 XP002229540 & SKIN RESEARCH, vol. 32, no. 4, 1990, pages 586-591, ISSN: 0018-1390
- DATABASE CA [Online] CHEMICAL ABSTRACTS SERVICE, COLUMBUS, OHIO, US; TABATA, YOSHIKO ET AL: "Method and two-component composition for straightening of curly hair" retrieved from STN Database accession no. 123:40687 XP002243246 & JP 07 101840 A (KAO CORP, JAPAN) 18 April 1995 (1995-04-18)
- AGNEW L.: 'Dorland's illustrated medical dictionary', 1965, W.B. SAUNDERS COMPANY, PHILADELPHIA, US * page 1044 *
- MEYERSON M.S. ET AL: 'Open-label study of the safety and efficacy of Fungoid tincture in patients with distal subungual onychomycosis of the toes' CUTIS vol. 49, no. 5, 1992, pages 359 - 362

## Description

### FIELD OF THE INVENTION

This invention relates to the use of a composition in the manufacture of a medicament for non-systemically treating nail fungal conditions, including onychomycosis.

### BACKGROUND OF THE INVENTION

Mammalian nails and hooves are high in keratin protein and can be denatured by the compositions of the invention. This denaturing of keratin leads to the softening of surface keratin and keratin debris, the buildup of nail tissue under the nail plate medically known as onychomycosis (OM), a localized infection of the nail or nail bed caused by pathogenic fungi. While prevalent in both finger and toe nails, it is primarily a disorder of the toenails occurring more often in older adults and constituting between 18% and 40% of all nail disorders. Also known as tinea unguium, OM is caused by microorganisms of the dermatophyte family, candida albicans and, rarely, the mold scopulariopsis brevicaulis. OM initially presents as a thickening and opacification of the nail plate edges. White patches may form where air pockets occur and various degrees of erosion, subungual hemorrhaging and other discoloration can result as the fungus advances. Total dystrophic OM, which is the most advanced and common form, causes the nail plate to become thickened with underlying keratin debris elevating the nail plate at a severe angle to the nail bed. Pain and difficulty in wearing foot apparel is often experienced.

### Current Methods of Treatment

Because dermatophytes are invasive to the keratin nail tissue, nail fungal infections are one of the hardest forms of external infection to treat. While current treatments are somewhat effective, they have adverse side-effects, and are contraindicated for patients taking certain drugs. Recently FDA-approved *Terbinafine* (Lamisil® ) is now the generally-accepted drug of choice. It is a synthetic allylamine compound that inhibits the action of squalene expoxidase, a crucial enzyme in the formation of ergosterol, leading to membrane disruption and dermatophytic cell death. Oral terbinafine is generally well-tolerated with the most common adverse effects being nausea, abdominal pain and allergic skin reactions. Taste disturbance and hepatic toxicity have also been reported. *Itraconzole* (Sporanox® ) is an alternative treatment which inhibits cytochrome P450 dependent synthesis of ergosterol. Reported side effects include headache, rhinitis, upper respiratory tract infection, sinusitis, reversible hepatitis, severe hepatotoxicity, diarrhea, dyspepsia, flatulence, dizziness, nausea, cystitis, urinary tract infection, myalgia, appetite increase, constipation, gastritis, gastroenteritis, myalgia, fever, pain, tremor, herpes zoster, asthenia, pharyngitis, rash and vomiting. Numerous drug interactions with itraconzole also cause plasma level increases and decreases. Clinical trials indicate that 89% of users noted improvement, 14% were cured and 21% of the cured group experienced reinfection. In the past, *griseofulvin,* an antifungal agent derived from a number of penicillium species inhibiting cell division and nucleic acid synthesis, *ketoconazole,* an oral or topical synthetic ioxolane imidazole compound which interferes with the biosynthesis of ergosterol, and *fluconazol,* an oral synthetic bistriazole compound that inhibits the cytochrome P450-dependent 14 alphademethylation step in the formation of ergosterol, were prescribed for OM and, for some patients, remain the drug of choice, despite a higher risk of severe side effects.

Like oral methods, topical medications for OM require continuing treatment over many (i.e., 3-18) months. Removal of the nail by surgical means or by 40% urea cream may hasten the total duration of treatment. Fungicidal creams include myconazole nitrate, clotrimazole, 10% povidine iodine and 1% econozole nitrate.

### Background of Weak Acids

Weak acids are acids which only feebly conduct electricity (low conductivity) and are only partially ionized in solution. The conductivity of solutions of acids has been thoroughly studied and the electrical conductivity of the weak acid, glacial or acetic acid (C₂H₃O₂H), for example, at a molarity of 0.1 is 4.67 reciprocal ohms (mho). This contrasts to the conductivity of strong acids which ionize completely in solution and have high conductivities ranging from 60 to 350 mho at the same molarity. Weak acids ionize only partially and their ions continue to react with each other, dissociating and recombining continuously in a condition known as the position of equilibrium. As is known, this occurs because the polar water molecules start to break the acids into its ions but cannot stop them from also being attracted to each other. While water molecules are sufficiently polar to prevent any permanent recombinations of the ions of strong acids, water is less effective at this task in handling weak acids and recombination of ions begins to take place as soon as any appreciable concentration of ions is present in the solution. Once the rate of recombination
catches up to the rate of dissociation both processes continue to proceed at the same rate and a state of equilibrium then exists.¹⁵
¹⁵Hess, Fred C., Revised by Thomas, Arthur L., *Chemistry Made Simple*, Rev. Ed 1984, Doubleday & Co, NY 1984, p. 71-74

It is further known that if 0.01 mole of pure acetic acid is dissolved in a liter of water at room temperature, about 4% of the solute will be ionized by the time equilibrium is reached. However, this position of equilibrium will vary depending upon the temperature and concentration. For example, if 0.1 mol of pure acetic acid is added to a flask so that the final volume is 1 liter, only 1.3% of the acetic acid is ionized to acetate.¹⁶ The remaining 98.7% remains in solution. The higher the percentage of weak acid, the stronger the denaturing properties of the solution by protonation of keratin's carboxylic amino acids. An increase in temperature will also shift the position of equilibrium in the direction of the process absorbing energy and make the acid more reactive with carboxylic amino acids. Thus the reactivity of the solution can be modulated by both the concentration of the solution and its temperature.
¹⁶*Organic Chemistry*, Ibid., p. 772.

Acetic acid (CH₃COOH) is a non-polar solvent (Subclass 102): 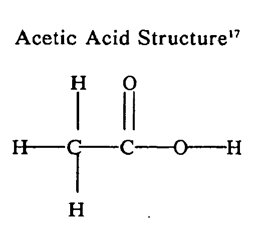
¹⁷Wede, L.G., Jr., *Organic Chemistry*, (Prentice Hall, 1987); See also Atkins P, *Atoms Electrons and Change,* WH Freeman & co., New York 1991, p.46

Acetic acid is identified as glacial acetic acid (in pure form), and in water solution as ethanoic acid, ethylic acid, methanecarboxylic acid, pyroligeneous acid, and vinegar acid.

For example, vinegar, a 5% aqueous solution of acetic acid, is produced by fermentation of sugars and starches. In fact, when fermented alcoholic beverages such as wine and cider are exposed to the air, the alcohol is converted to acetic acid.

Acetic acid is used in the manufacture of acetic anhydride, cellulose acetate, vinyl acetate monomer, acetic esters, chloroacetic acid, as well as the production of plastics, pharmaceuticals, dyes, insecticides, photographic chemicals, etc.; it is also a food additive (acidulant), a latex coagulant, oil-well acidizer and is used in textile printing.¹⁸ Acetic acid is recognized in the art as a fixing agent of protein, capable of preserving both the structure and/or chemical composition of animal or plant tissues without combining with or precipitating any proteins.¹⁹ Acidic acid solutions for the purification of proteins include methods to purify fragments of fibrinogen important to blood-clotting.²⁰ It is also known in the practice of biochemistry peptide sequencing that disulfide bonds can be oxidized *in vitro* by the carboxylic acid, performic acid,²¹ which converts all *cys* residues -- whether linked by disulfide bridges or not -- to cysteic acid residues that are stable in both acidic and basic solutions. The vulnerability of disulfide bonds to acetic acid attack has also been utilized in foodstuff processing²² and cheese manufacture.
¹⁸Hawley, Gessner G., *The Condensed Chemical Dictionary,* 9^{th} Ed., Van Nostrand Reinhold Co, New York, 1977, p.5
¹⁹Four types of fixing agents are possible: (1) Additive, coagulant, (2) additive, non-coagulant, (3) non-additive, coagulant, and (4) non-additive, non-coagulant Other major fixing agents defined by category are: Acetone (3), chromium trioxide (1), ethanol (3), formaldehyde (2), glutaraldehyde (2), mercuric chloride (1), methanol (3), osmium tetroxide (2), picric acid (1), potassium dichromate (2) and trichloracetic acid (3).
²⁰The Euglobulin Clot Lysis time test (CPT code: 85360) uses 10% acidic acid solution to precipitate fibrinogen prior to mixing it with thrombin to measure clot lysis time.
²¹Source: Biochemistry 659: Class Notes, Perdue University, 1967.
²²Keck-Gassenmeier B., Wieser H. (1996), *Disulfide bonds in acetic acid: Soluble and insoluble glutenin fractions*. In: Gluten '96, Proceedings of the 6^{th} International Gluten Workshop (Wrigley C.W., ed.) RACI, North Melbourne, Australia, pp.145-148.

U.S. Patent No. 4,240,450 to Grollier, *et al*., describes the compositions having a combination of an anionic polymer and a cationic copolymer containing sulfonic acid, carboxylic acid or a phosphoric acid end unit, wherein the compositions are said to be useful for treating keratin materials.

U.S. Patent No. 5,091,171 to Yu, *et al*., describes the use of amphoteric compositions containing alpha hydroxyacids, alpha ketoacids, related compounds or polymeric forms of hydroxyacids in the treatment of dermatologic disorders including nail infections.

U.S. Patent No. 2,729,586 to Rystan Company, describes the use of water soluble chlorophyll and salts of fatty acids in the treatment of dermatomycoses.

U.S. Patent No. 5,462,714 to Talwalker, R.T. and Barve, S.S. describes the use of a combination of iodine, fatty acid, non-ionic surfactant, buffering agent and water as a substantially non-corrosive antimicrobial composition.

E.P. Patent Application No. 0755676 A1 describes the use of a composition comprising a monocarboxylic acid as a keratin hardening agent.

Patent Application No. WO/99/20250 describes the use of a topical, foamable composition containing at least one antifungal agent including fatty acids, and having less than 20% of a nonvolatile component in the treatment of fungal diseases.

Meyerson, M.S. *et.al*., (1992) Cutis 49 (5), 359-362 describes a study using topical Fungoid® Tincture in the treatment of onychomycosis.

### SUMMARY OF THE INVENTION

The invention relates to the use of a composition in the manufacture of a medicament for denaturing keratin for non-systemically sloughing or exfoliating fungus-infected keratin of the toe and fingernail.

### BRIEF DESCRIPTION OF THE DRAWINGS

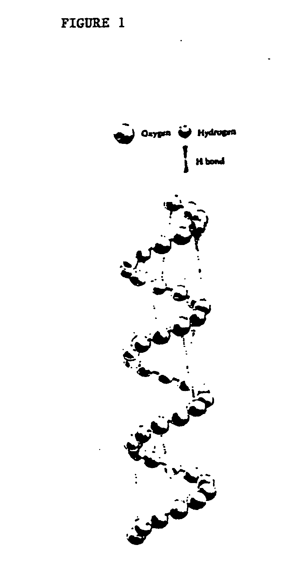

Figure 1 shows the rubberband-like hydrogen bonds of α-helix keratin coils.
Figure 2 shows the results of treatment of onychomycosis and hyperkeratosis. The photographs show the patient's generalized callous and OM on large toenail prior to and after treatment as discussed in Example 1. This example also shows hyperkeratosis (reference) and callouses (pre and post-treatment) (reference) in the patient.

### DETAILED DESCRIPTION OF THE INVENTION

The invention relates to the use of a composition in the manufacture of a medicament for denaturing keratin for non-systemically sloughing or exfoliating fungus-infected keratin of the toe and fingernail.

This is achieved by regular and periodic bathing of the keratin tissue in an aqueous solution containing alkanoic acid.

Thus, the invention provides a use of a composition comprising between 1% and 20% by weight of at least one alkanoic acid in aqueous solution selected from the group consisting of acetic acid, methanoic acid, propanoic acid, decanoic acid, octanoic acid, heptanoic acid, hexanedioic acid, and octadecanoic acid and a suitable carrier, in the manufacture of a medicament for the non-systemic treatment of nail fungal conditions.

The preferred alkanoic acid is acetic acid (CH₃COOH) at 5% solution (aq) with water as the diluent.

Another alkanoic acid, formic acid (methanoic acid, HCO₂H), while very low in pH (2.37) may be substituted for or added to the preferred weak acids, propionic acid and acetic acid, in bath or lotion formulation. For nail application, the recommended concentration is 2.0 mol. of formic acid to one liter of solution.

The term "diluent" as used herein refers to substances used to dilute the respective acids. Suitable diluents include water and a variety of alcohols.

### Softening of Nail Tissue

The uses described herein gradually soften, dissolve and permit excoriation of the surface and excess nail tissue known as keratin debris, in which fungi survive. By attacking certain amino acids of the affected keratin debris, it becomes softened, permitting its safe and painless removal, gradually and over time, while adjacent healthy tissue which contains less keratin is left unaffected by the treatment.

Acidic and propionic (propanoic) acids are known to have antifungal properties, particularly against pseudomonas *aeruginosa, candida* and *aspergillus* strains. The use of these acids for the treatment of nail fungal diseases, however, has not been effective as these acids cannot be safely applied to nail tissue at high strengths, and cannot penetrate to the source of the fungus which lies subungually below the nail and within the hard keratin debris.

It should be noted that while the presence of an alkanoic acid in a low concentrate aqueous solution may enhance the antifungal results of the method in treating OM, this invention treats keratin, the *habitat* of the fungus. By eradicating that habitat, as a sloughing agent, the fungus itself is eliminated. This is similar to the current research exploring anti-angiogenic agents which prevent cancer tumors from sustaining a vascular system. By selectively eliminating the tumor's environment, it dies. Similarly, OM is treated by dissolving the specific keratin tissue in which OM fungi survive.

Additionally, when the use disclosed herein is followed, the known anti-fungal properties of acetic and propanoic acids are effectively delivered to the nail fungal habitat allowing them to come into contact with the fungal strains and act as fungicides. This is achieved by the electrostatic action triggered by the inability of alkanoic acids to ionize in water and protonate the carboxylic amino acids of keratin protein. The invention is thus a safe and effective, non-systemic treatment of nail fungal conditions by a novel way of delivery of a known fungicide, and denaturing of keratin which dissolves and sloughs as a consequence of the protonation of the two carboxylic amino acids in keratin, glutamic acid and aspartic acid which, in turn, leads to the denaturing of cystine and the resultant cleavage of disulfide bonds. It is this pathway of events which results in the physical manifestation of softened keratin tissues. Once softened after several weeks of soaking treatments, high keratin-containing tissue can be safely lifted from normal tissue by dissolving, scrapping or excoriation. In OM, fungus is no longer present as a consequence of the removal or sloughing of its environment.

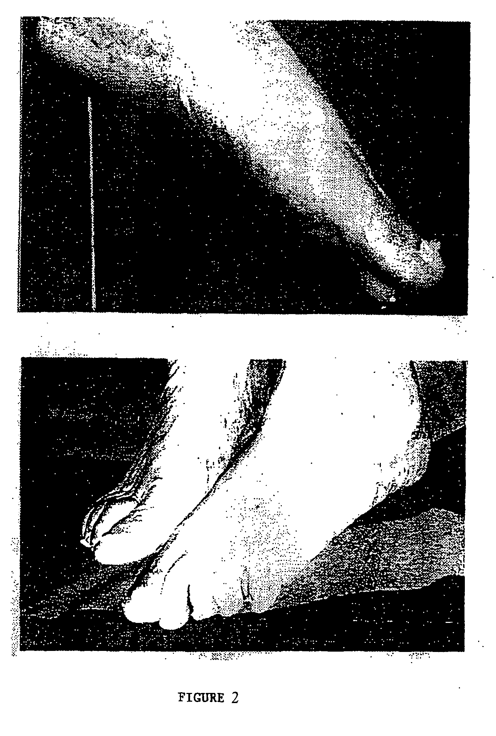

The uses described herein gradually soften, dissolve and permit excoriation of the excess nail tissue in which the fungi collect and survive. By attacking the keratin debris of the nail and permitting its safe and painless removal, gradually and over time, the remaining tissues -- healthy nail and normal, or soft skin -- containing lower levels of keratin, are left unaffected by the treatment and, in the case of OM, fungus-free. The uses described herein exposes the nails to a one to three month regimen of twice daily foot baths in which an alkanoic acid is dissolved in warm water at a preferred ratio of one part acid to 16 parts warm water. The electrostatic action of the weak acid in water (causing protonation of glutamic acid and aspartic acid within keratin protein and the cleavage of its disulfide bonds) gradually softens the keratin of the nail. Keratin debris softens at a greater rate than the healthy nail plate, resulting in its dissolution and easy removal by mechanical means. Discoloring fungus on the surface and edges of the nail plate is also dissolved along with surface nail plate cells, leaving the nail clear and topically fungus free. Simultaneously, the non-ionized alkanoic acid electrostatically attacks the carboxylic amino acids within subungual keratin debris softening it until eventually the base, lumina area, is exposed and debris dissolved from this area as well. As the keratin debris softens the treatment can be hastened by gently boring into the keratin debris with a small nail cuticle tool or pick and creating irrigation channels through which the acid bath solution can penetrate to further speed the softening process. Without an environment in which to survive, the fungus itself disappears. The process takes from one to three months depending on the amount of existing debris material to be dissolved, softened and excoriated (see Example 1 and Figure 2).

A similar procedure and composition can be used for a hand bath in which fingernails contaminated with fungal infection can be treated.

### Mechanism of Action

While not wishing to be bound by any one theory, it is believed that the present compositions are capable of gradually and by repeated applications altering the secondary structure of keratin's polypeptide chain by reacting with the carboxylic acids and in turn the amine bases of this protein, such that glutamic acid and aspartic acid are protonated with their electron charge changing from negative to neutral/positive. This reaction is believed to result in the following denaturing events:
1. Protonation of glutamic acid and aspartic acid is known to cause these amino acids to turn inward on themselves or "inside out", changing at these locations the hydrogen bonding distances and strength.
2. Salt bridges are also cleaved whereby aspartic and glutamic acid electron charge change. As is known, these negatively-charged amino acids form ionic bonds, also know as salt-bridges, with arginine and lysine which are positively charged. When the former lose their charge the ionic bonds cleave and the keratin is further denatured.
3. Disulfide bonds "let go", it is thought, as protonation deconfigures the protein's secondary structure. These bonds are still in place but no longer anchored on the chain.

Once a percentage of the ionic, hydrogen and cysteine bonds are cleaved by repeated rinsing with the weak alkanoic acid solutions, keratin in nail tissues will soften and eventually slough.

The compositions may further comprise a lotion, creme, gel, or ointment, or the carrier itself may be a lotion, creme, gel or ointment. The compositions may further comprise a humectant or thickening agent.

In a preferred embodiment, the invention relates to the use of compositions for the manufacture of a medicament for the treatment of nail fungal conditions comprising at least one alkanoic acid in aqueous solution, wherein the ratio of said alkanoic acid to water is between 1:16 and 1:20.

### Example 1 - Treatment of Onychomycosis and Hyperkeratosis (reference)

A 76 year old male patient who reported suffering from onychomycosis on all toe nails for 30 years as well as generalized foot callouses was selected for treatment. Both large toe nails had lifted at a 45° angle off the nail bed, being compacted subungually with keratin debris. The patient also suffered from a generalized callous along the outer lateral edges of the soles of each foot. He was placed on a twice daily, 5 minute soak, foot bath regimen for eight weeks. After two weeks, the keratin debris had softened on the outer edges and could be irrigated by boring with a fine instrument to permit greater exposure to the weak acid bath solution. After four weeks the yellow color of the nail surfaces had resolved to clear and the patient's large toe nails began to flatten as keratin debris continued to slough. At the end of six weeks, much of the fungus had disappeared in tandem with the loss of keratin debris and the callouses had pealed off revealing soft skin beneath. The small toe nails appeared completely rejuvenated. At eight weeks, the patient's toe nails were free of both keratin debris and the fungus they harbored and new nail growth was adhering to the nail bed (see Figure 2).

### Example 2- Delivery of Antifungal Agents to Infected Nail Tissue

To a solution composed of 1:16 dilute alkanoic acid/water concentration, water-soluble fungicides such as terbinafine hydrochloride or fluconazole (slightly soluble), or fungistats, such as griseofulvincan, are added to nail fungal bath treatment as described herein. The addition of known fungal agents heightens the attack on dermatophytes or other fungal organisms within hardened nail keratin and keratin debris. As the acid/water baths continue to soften, slough and expose new keratin areas the habitating fungus are killed or rendered static by the fungicide or fungistat bath ingredient. This treatment may include the use of one particular alkanoic acid, acetic acid, which is a known fungicide. A topical fungicide or fungistat, such as terbinafine hydrochloride cream, myconazole nitrate, clotrimzole, 10% providine iodine and 1% econozole nitrate, may be applied between alkanoic acid/water baths and after manual excoriation of the softened nail areas to hasten the therapeutic response. This delivery or neoadjuvant treatment is also effective against ringworm and other fungal infections of the nail and hyperkeratotic skin.

## Claims

1. Use of a composition comprising between 1% and 20% by weight of at least one alkanoic acid in aqueous solution selected from the group consisting of acetic acid, methanoic acid, propanoic acid, decanoic acid, octanoic acid, heptanoic acid, hexanedioic acid, and octadecanoic acid and a suitable carrier, in the manufacture of a medicament for the non-systemic treatment of nail fungal conditions.

2. Use according to claim 1 wherein said alkanoic acid in aqueous solution comprises acetic acid.

3. Use according to claim 1 or claim 2 wherein said composition further comprises at least one of a lotion, creme, gel, or ointment.

4. Use according to any of claims 1 to 3 wherein said composition further comprises at least one of a humectant and a thickening agent.

5. Use according to any of claims 1 to 4 wherein said alkanoic acid in aqueous solution comprises acetic acid present in an amount between 5% and 20% by weight of the composition with water as a diluent.

6. Use according to any of claims 1 to 5 wherein said nail fungal condition is onychomycosis.

## Patentansprüche

1. Verwendung einer Zusammensetzung, die zwischen 1 Gew.% und 20 Gew.% von mindestens einer Alkansäure in wässriger Lösung umfasst, ausgewählt aus der Gruppe bestehend aus Essigsäure, Ameisensäure, Propionsäure, n-Dekansäure, Oktansäure, Heptansäure, Adipinsäure und Oktadekansäure und einem geeigneten Träger, für die Herstellung eines Medikaments für die nicht-systemische Behandlung von Nagel-Pilz Erkrankungen.

2. Die Verwendung gemäß Anspruch 1, wobei Alkansäure in wässriger Lösung Essigsäure umfasst.

3. Die Verwendung gemäß Anspruch 1 oder Anspruch 2, wobei die Zusammensetzung zudem mindestens eine Lotion, eine Creme, ein Gel oder eine Salbe umfasst.

4. Die Verwendung gemäß einem der Ansprüche 1 bis 3, wobei die Zusammensetzung zudem mindestens ein Befeuchtungsmittel und ein Verdickungsmittel umfasst.

5. Die Verwendung gemäß einem der Ansprüche 1 bis 4, wobei die Alkansäure in wässriger Lösung Essigsäure in einer Menge zwischen 5 Gew.% und 20 Gew.% der Zusammensetzung mit Wasser als Verdünnungsmittel umfasst.

6. Die Verwendung gemäß einem der Ansprüche 1 bis 5, wobei die Nagel-Pilz Erkrankung die Nagelmykose ist.

## Revendications

1. Utilisation d'une composition comprenant entre 1 % et 20 % en poids d'au moins un acide alcanoïque dans une solution aqueuse choisi dans le groupe constitué par l'acide acétique, l'acide méthanoïque, l'acide propanoïque, l'acide décanoïque, l'acide octanoïque, l'acide heptanoïque, l'acide hexanedioïque et l'acide octadécanoïque et au moins un véhicule adapté, dans la fabrication d'un médicament destiné au traitement non systémique d'états fongiques de l'ongle.

2. Utilisation selon la revendication 1, dans laquelle ledit acide alcanoïque dans une solution aqueuse comprend l'acide acétique.

3. Utilisation selon la revendication 1 ou la revendication 2, dans laquelle ladite composition comprend en outre au moins une lotion, une crème, un gel ou une pommade.

4. Utilisation selon l'une quelconque des revendications 1 à 3, dans laquelle ladite composition comprend en outre au moins un humidifiant et un épaississant.

5. Utilisation selon l'une quelconque des revendications 1 à 4, dans laquelle ledit acide alcanoïque dans une solution aqueuse comprend de l'acide acétique présent dans une quantité comprise entre 5 % et 20 % en poids de la composition avec de l'eau comme diluant.

6. Utilisation selon l'une quelconque des revendications 1 à 5, dans laquelle ledit état fongique de l'ongle est l'onychomycose.
